# EUROPEAN PATENT APPLICATION

(11) **EP 3 323 360 A1**
(43) Date of publication of application: **23.05.2018**
(21) Application number: 17275158.8
(22) Date of filing: 05.10.2017
(51) Int. Cl.: A61B 17/12, A61F 2/01, A61F 2/86, A61F 2/07

(54) **IMPLANTABLE MEDICAL DEVICE WITH ATRAUMATIC TIP**

(30) Priority: 21.11.2016 GB 201619644
(71) Applicant: Cook Medical Technologies LLC, Bloomington, IN 47404 (US)
(72) Inventor: BUTZBACKER, Raimo Urban, 4690 Haslev (DK); ELGAARD, Per, 4690 Haslev (DK)
(74) Representative: Williams Powell

(57) **Abstract**

An implantable medical device (10) includes an expandable support structure (12), which may be a stent. In the example shown, the device is a vascular plug and includes a twisted occluding sleeve (30) held within the stent (12), which closes the lumen through the stent (12). At a distal end (18) of the stent (12), there is provided a guide tip (50) made of a resilient material, which includes a plurality of separable legs 62 coupled to a central hub (64). The legs (62) extend radially outwardly when the device (10) is in its radially expanded configuration, providing an open passage to the medical device (10). This structure of combined medical device (10) and expandable guide tip (50) enables the omission of a conventional dilator tip of an introducer assembly and allows vascular plugs and similar devices to be deployed over-the-wire and with the assistance of a dilator tip, while avoiding having to pull the dilator tip through the deployed medical device after implantation.

## Description

### Technical Field

The present invention relates to an implantable medical device having an atraumatic tip connected to the device. The medical device can be any device deployed in an organ of a patient, such as in the patient's vasculature, and is particularly suitable for devices which in the deployed configuration do not have a sizable lumen passing therethrough. Examples include vascular plugs, filters and so on. The teachings herein are not, though, restricted to such devices and can equally be used with tubular stents, stent grafts and other similar devices.

### Background Art

There is a significant industry in the provision and use of implantable medical devices. These are conveniently deployed endoluminally from a remote percutaneous entry point, such as the femoral artery of jugular vein, to cite just two examples. Such a procedure often involves the well-established Seldinger technique and utilises an elongate introducer assembly. A guide wire may be used in the procedure, which is positioned in the patient's vasculature up to the location of the intended treatment site. The introducer assembly includes an elongate device carrier which is housed in an outer, protective, sheath. At a distal end of the assembly the is provided a soft tip, typically a dilator tip, which assists in guiding the introducer assembly through the patient's vasculature and in particular to ensure that the introducer assembly curves around curved vasculature and/or passes into bifurcations or side branches. The dilator tip is typically connected to an inner catheter or pusher element of the assembly, normally also the device carrier, and is removable with the inner catheter once the medical device has been deployed. As the dilator tip is located at the extremity of the introducer assembly and distally of the medical device carried on the assembly, the dilator tip must pass through the deployed medical device on withdrawal of the introducer assembly at the end of the medical procedure. For this purpose, there needs to be a sufficient passage through an interior lumen or through the outside of the deployed medical device. In many instances this does not pose a problem, such as in the case of tubular devices such as stents and stent grafts. However, in the case of a medical device not having a sizeable lumen extending therethrough, such as a vascular plug or filter, difficulties arise with the removal of such a dilator tip. Omitting the dilator tip from the assembly may not be an option as it can lead to a reduction in the assembly's effectiveness and can prevent its deployment in highly curved vessels.

Some examples of known publications relating to medical devices, particularly occlusive medical devices and their methods of deployment, can be found in WO-02/00139, US-2003/0171771, US-6,080,183, US-5,401,257, US-2012/0296256 and US-2009/0112062.

### Summary of the Present Invention

The present invention seeks to provide an improved implantable medical device and in the preferred embodiment an improved vascular occluder. The present invention is also directed to an introducer assembly including an implantable medical device carried thereon, the medical device having the features disclosed herein.

According to an aspect of the present invention, there is provided an implantable medical device having an expandable support structure, the support structure including a proximal end and a distal end;
a guide tip of flexible material and having a proximal end and a distal end, the guide tip including a plurality of sections extending from the guide tip distal end to the guide tip proximal end, the sections being attached to the distal end of the support structure and being radially expandable with the support structure, thereby allowing passage of fluid through the guide tip at least when the support structure is in an expanded condition and the sections radially expanded.

The structure is such that the guide tip, typically a dilator tip, is integral with the medical device and is not fixed to the introducer assembly. As a result, the guide tip remains in the patient. The provision a guide tip which allows passage of blood therethrough when the medical device is deployed ensures that the normal function of the medical device is not affected by the presence of the guide tip.

In the preferred embodiment, the distal end of the guide tip retains a substantially constant diameter when the support structure is expanded. The advantage of this structure is that the guide tip retains its distal end configuration, which can optimise the integrity and positioning of the assembly when in a patient.

In a practical embodiment, the guide tip is formed of a plurality of longitudinally separable sections at least at the proximal end thereof. As the sections separate from one another on expansion of the medical device, fluid can pass through the guide tip to the medical device to enable the medical device to function as desired.

The guide tip may be formed as a plurality of longitudinally separate sections at its proximal end, said sections being connected together at the distal end of the guide tip.

Remaining connected at the distal end ensures that the guide tip retains a generally singular configuration and in practice can prevent separate sections from moving or otherwise behaving independently of one another. In some designs this may exhibit itself as flapping of sections in the flow of blood, which could cause premature wear and possible interference with the flow of blood or functioning of the medical device.

Advantageously, the guide tip is self-supporting. In the preferred embodiments, the guide tip is not provided with any support frame along the length thereof; in other words does not have any support struts within or coupled to the separable or separate sections of the guide tip.

In a preferred embodiment, the guide tip includes a passage extending therethrough. Typically, this is for receiving a guide wire.

Preferably, the guide tip has a shape tapering from its proximal end to its distal end. The guide tip is preferably at least partially frustoconical. In other words, the guide tip may be a dilator tip.

The support structure is preferably an expandable frame formed of a plurality of struts, with the proximal end of the guide tip being attached to one or more the struts or to fixation points thereof.

In a practical embodiment, the support structure is an expandable stent.

In some embodiments the medical device is a vascular plug, occluder or filter, in which case the support structure may support an occlusion or filter member. The teachings herein are, however, not limited to these types of medical devices only and could be used with any implantable medical device. Even in the case of devices which created a sizeable lumen within the zone of the device, such as a stent or stent graft, the guide tip taught herein can still provide notable advantages, such as avoiding having to retract the dilator tip through the device once deployed, with the consequential risks of snagging of the dilator tip against the device.

Preferably, the support structure is self-expanding. It may be formed of a shape memory material, such as nickel titanium alloy, or a spring material such as spring steel.

According to another aspect of the present invention, there is provided an introducer assembly including a carrier catheter having a proximal end and a distal end, and a lumen extending therethrough;
an implantable medical device having an expandable support structure, the support structure including a proximal end and a distal end;
a guide tip of flexible material and having a proximal end and a distal end, the guide tip including a plurality of sections extending from the guide tip distal end to the guide tip proximal end, the sections being attached to the distal end of the support structure and being radially expandable with the support structure, thereby allowing passage of fluid through the guide tip at least when the support structure is in an expanded condition and the sections radially expanded;
wherein the implantable medical device is disposable at the distal end of the carrier catheter with the guide tip at least partially extending beyond the distal end of the carrier catheter.

There is preferably also provided an elongate carrier element, the implantable medical device being disposed on the elongate carrier element for insertion into the carrier catheter. The elongate carrier element may be a guide wire catheter.

Preferably, the assembly includes a protective sheath disposed over the implantable medical device, the guide tip being positionable beyond a distal end of the protective sheath to act as a dilator tip.

Other features and advantageous of the invention taught herein will be apparent from the following description and accompanying drawings.

### Brief Description of the Drawings

Embodiments of the present invention are described below, by way of example only, with reference to the accompanying drawings, in which:
Figure 1 is a side elevational view of a preferred embodiment of implantable medical device according to the teachings herein;
Figure 2 is an enlarged view of a distal end of the medical device of Figure 1 showing the expandable guide tip;
Figure 3 is a distal end view of the medical device of Figure 1;
Figure 4 is a side elevational view of the distal end of the device of Figure 1 in a radially compressed configuration;
Figure 5 is a side elevational view in partial cross-section of an introducer assembly including a medical device according to Figure 1 carried therein;
Figure 6 is an enlarged view of the distal section of the assembly of Figure 5;
Figure 7 is a side elevational view of the assembly of Figure 5, also in partial cross-section, showing the primary elements of the assembly; and
Figure 8 is a side elevational view of the distal end of the introducer assembly showing the medical device in the process of being deployed form the assembly.

### Description of the Preferred Embodiments

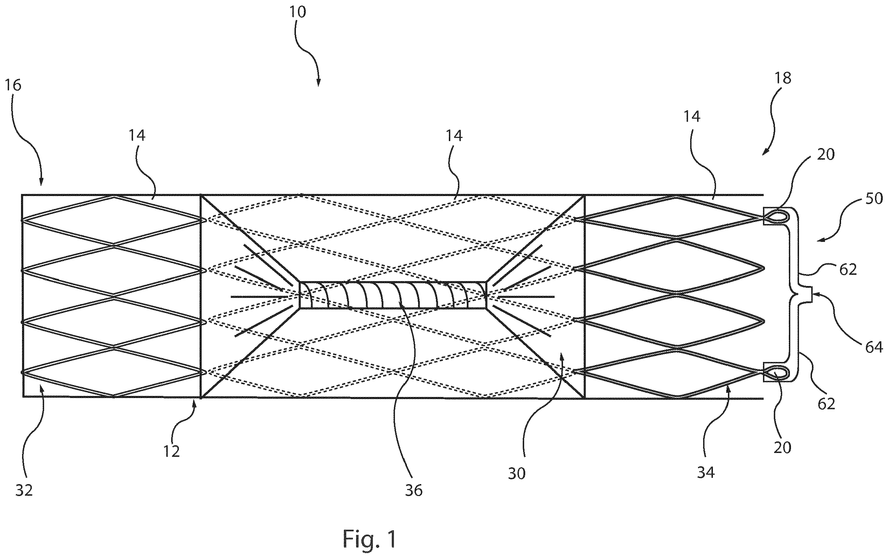

The preferred embodiment described below is configured as a vascular plug, or occluder. It is to be understood, though, that the teachings herein are applicable to a wide variety of implantable medical devices, including other occlusion devices and filters, such as conical and hourglass shaped occluders and filters, stent, stent grafts and so on. The structure shown in Figure 1, although configured as a vascular plug, could equally be structured as a filter, for instance by use of a permeable filter mesh rather than an occluding sleeve.

In this specification, the terms "proximal" and "distal" are used consistently and are referenced by the relative positions of the introducer assembly when used by a physician. Thus, the term "proximal" refers to a position closest to the physician and the term "distal" is further from the physician. In practice, the proximal end of the introducer assembly will be kept outside the patient and will include the manipulation tools, such as handles, ports, valves and so on, while the distal end will be positioned within the patient's vasculature, up to the position of the site to be treated. The same nomenclature is used in connection with the medical device per se; in other words, it is used to refer to the arrangement of the device with reference to its orientation when held in the introducer assembly.

Referring first to Figure 1, this shows an implantable medical device 10 in the form, in this embodiment, of a vascular plug or occluder. The device 10 includes a tubular support element 12, this being shown as a stent formed of a plurality of zigzag stent rings 14 connected in this example to apices or troughs of adjacent stent rings 14. The support element 12 is substantially cylindrical and round in transverse cross-section, although in other embodiments could have a different shape, for instance a tapering form or with a central waist. The stent rings 14 extend in this embodiment for the whole length of the tubular support element 12, the rings 14 in the central region being shown in dotted outline only for ease of visualisation of the interior contents of the device 10. The support element 12 in this embodiment could be the applicant's Zilver^{RTM} stent.

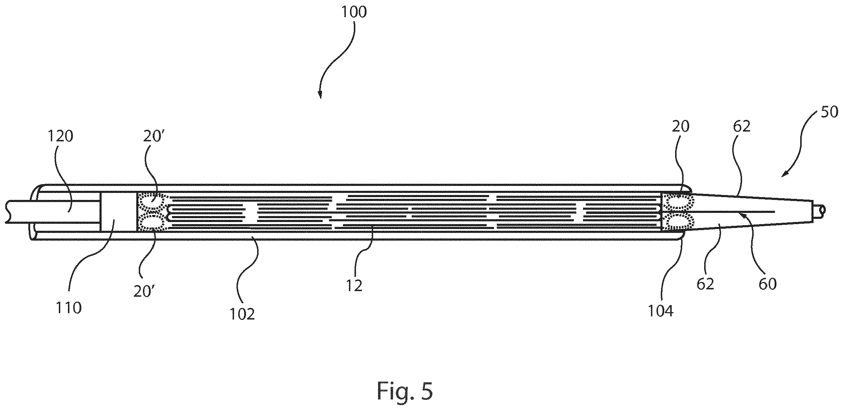

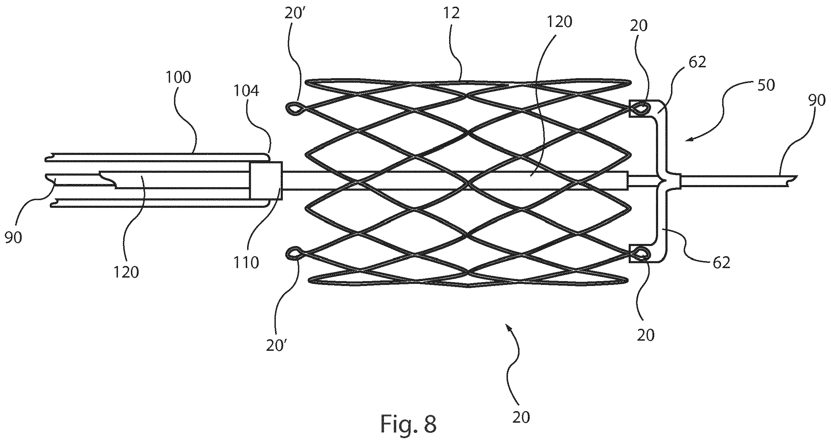

The support element 12 has a proximal end 16 and a distal end 18. As shown in Figure 1, the element 12 is provided with a plurality of radiopaque markers 20, which in this embodiment are disks of gold fixed into associated rings integral with the stent ends. Radiopaque markers of this type are known in the art. The proximal end 16 of the device 10 may also be provided with radiopaque markers similar to those shown at the distal end 18 in Figure 1 and indeed are shown in Figures 5 and 8.

The support element 12 is preferably made of a self-expanding material such as spring steel or a shape memory material such as a shape memory alloy. Nitinol is suitable.

The device 10 also includes a sleeve 30 disposed within the internal lumen of the tubular support element 12. The sleeve 30 has sleeve ends 32 and 34, which in this embodiment are wrapped over and to the outside of the support ends 16, 18. The wrapped-over sleeve ends 32 and 34 can usefully be bonded to the internal sleeve parts in the zone where they overlap, by suitable bonding material or fusion for instance, thereby fixing the sleeve 30 to the support element 12. In other embodiments, the sleeve ends 32, 34 could be sewn to the parts of the sleeve they overlap or could be attached directly to the frame of the support element 12, for instance to the stent rings 14, by sutures or the like.

The sleeve in this embodiment is made of an impermeable material such as ultra-high molecular weight polyethylene, for instance Dyneema^{RTM} or expanded polytretrafluoroethylene (EPTFE), polyester or any other known or suitable graft material. The material is preferably non-elastic but in some embodiments could be of elastic material, for instance having a maximum expansion of 5% at operational stresses.

The sleeve 30 could also be made of a material which is not completely impermeable but which is substantially impermeable, that is of a nature and structure that it will provide a sufficient barrier to blood flow to cause blood statis at the location of the device 10 and as a result cause the formation of thrombi which will occlude the vessel. In the preferred embodiment, though, the sleeve is of a material which will provide instantaneous occlusion of the vessel, so as to act as a near instantaneous plug.

The sleeve 30 may be made of woven or knitted material, or even sheet material. As will be apparent, the sleeve 30 is made of a thin sheet of material which is flexible so as to be turned into a sleeve and readily twisted on itself by a number of turns.

As shown in Figure 1, the sleeve is twisted along its length, causing it to constrict at location 36, typically around the midpoint along the supporting stent 12. This twist can be achieved by rotating the ends 32, 34 of the sleeve relative to one another during the fitting of the sleeve 30 to the support element 12. Typically, the ends will be rotated at least 360 degrees from one another, preferably 720 degrees or more. The constricted zone 36 preferably has a substantial length, which can ensure good occlusion of the lumen through the sleeve 30 and can be more effective than a short occluding barrier.

The sleeve 30 preferably has a diameter about the same as the diameter of the tubular support 12, although in some embodiments could be slightly larger. The length of the sleeve 30 extending within the tubular support 12 between its ends 16 and 18 is preferably longer than the length of the support 12, preferably at least 5 percent longer and even up to 40 to 50 percent longer in some cases.

The twist of the sleeve between its two ends 32 and 34, closes the lumen of the sleeve and therefore acts as a barrier. When the portion of sleeve 30 inside the support structure 12 is longer than the support structure itself, the extra length of sleeve causes it to gather together at the point of twist, that is at the waist produced by the twist. This enhances the closure of the lumen of the sleeve. The sleeve material will be caused to gather together particularly from the pressure of blood in the vessel, which will push the material against itself. Specifically, the pressure of blood against the upstream facing sleeve end 32 or 34 will push back the material of the sleeve in a downstream direction, thereby compressing the material at the location of the waist. The blood pressure will also create a parting force on the wall of the sleeve at the opening of the device 10, which will act to tighten the twisted part of the sleeve. Thus, blood pressure will act to maintain closing pressure on the sleeve, thereby avoiding or reducing the risk of recanalization.

When the support element 12 is radially compressed, for instance when it is loaded onto a carrier catheter of an introducer assembly, the radial compression of the sleeve 30 will loosen any radial tightening, allowing opening of the lumen of the sleeve, thereby allowing the passage therethrough of a guide wire catheter or other device.

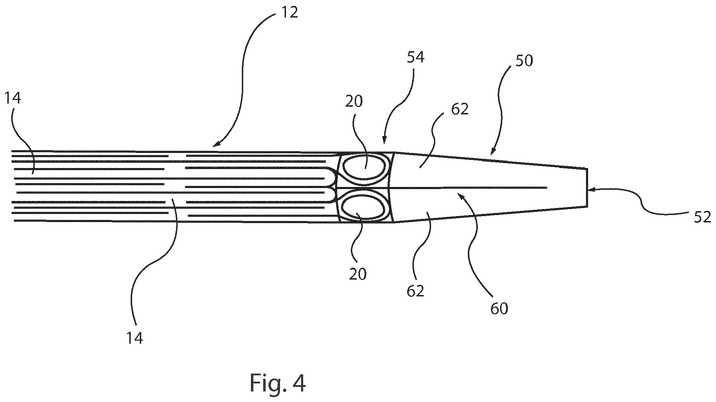

With reference now to Figures 1 to 4, at the distal end 18 of the support structure 12 there is provided a guide tip 50, which in Figures 1, 2 and 3 is shown in a radially expanded extended configuration, while in Figure 4 is shown a pre-expanded, radially compressed, configuration.

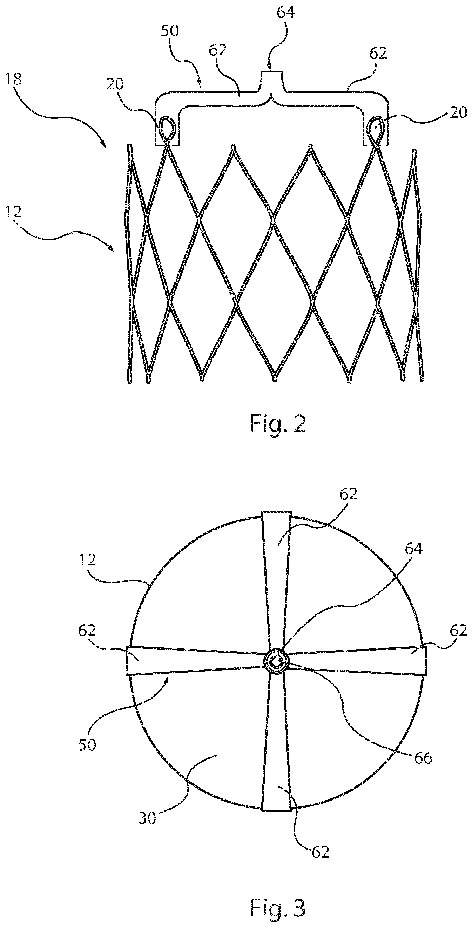

Considering Figure 4 first, when the support structure 12 is radially compressed, in the form in which it would be when disposed on an introducer assembly, the guide tip 50 has a frusto-conical shape with a distal end 52 and a proximal end 54. The radiopaque markers 20 of the stent 12 are embedded in the material of the guide tip 50 so as to be integral therewith. The guide tip 50 tapers from its proximal end 54 towards it narrower, distal end 52. In this configuration, the guide tip 50 has a lumen (not visible in Figure 4) which extends through its length, for receiving a guidewire or guidewire catheter, as will be shown in later drawings described below. Furthermore, the guide tip 50 includes a plurality of slit lines 60 extending from a position just proximal of the distal end 52 all the way to and including the very proximal end 54 of the tip 50. In this embodiment, there are four slit lines 60 substantially equally spaced radially around the guide tip 50. The slit lines 60 may be pre-cut through the thickness of the material forming the guide tip 50 or may be weakened zones designed to tear when stretched. As can be seen in Figure 3 in particular, which is a front elevational view of the medical device 10 in a radially expanded configuration, the four slit lines 60 delineate four arms of separable sections 60 of the guide wire tip 50, which extend from the proximal end 52, which forms a singular hub 64. In cases where there are more than four radiopaque markers (or less than four) on the medical device, the number of legs 62 could vary in accordance with the number of radiopaque markers and/or could be attached to only some of the radiopaque markers or, in the alternative, in part to the radiopaque markers and in part to other elements of or associated with the support structure 12.

The guide tip 60 is preferably made of a soft polymer material, such as silicone, a polyether block amide (such as Pebax^{RTM}), a polyamide (such as nylon), polyurethane, or any other suitable material.

The provision of a soft guide tip 50 enables this to be used in place of a conventional dilator tip of an introducer assembly.

The guide tip 50 is preferably self-supporting. In the preferred embodiments, the guide tip 50 is not provided with any support frame along the length thereof; in other words does not have any support struts within or coupled to the sections 62 of the guide tip 50. Most preferably, the guide tip, or at least the legs 62, is formed solely of soft polymer material.

Comparing now Figure 4 with Figures 1, 2 and 3, when the support structure 12 of the medical device 10 radially expands, as it will do when deployed in a patient's vessel, the expansion of the anchor points attached to the extremities of the legs 62, in this example the radiopaque markers 20, will pull at the extremities of the legs 62 causing them to separate from one another along the slit lines 60. Where the slit lines are lines of weakness, their separation will be by splitting the material of the guide tip 50. In other examples, for instance when the legs are held to one another by adhesive, this will be by rupturing the adhesive bond. It is preferable that some form of connection or bond between the adjacent legs 62 is broken in this process, although in some embodiments the legs may already be separate and simply line alongside one another in the radially compressed configuration of Figure 4.

As the legs 62 separate from one another, the guide tip 50 will contract longitudinally, as will be apparent from a comparison of Figure 4 with either one of Figures 1 and 2, for example. As the result of this, the guide tip 50 will not extend much beyond the longitudinal extent of the support structure 12, which delimits the volume of the operative part of the medical device. This longitudinal contraction minimises any impact of the guide tip 50 on the patient's vasculature, or other organ, beyond the location of the support structure 12 of the medical device.

Furthermore, as will be particularly apparent from Figures 1, 2 and 3, the opening of the legs 62 will provide virtually unrestricted access to the main structure in the medical device 12, in this embodiment to the vascular plug and in the particular the twisted sleeve 30 which acts as that plug. In practice, the arrangement is such that the guide tip 50, when so expanded, will not interfere with the normal functioning of the medical device.

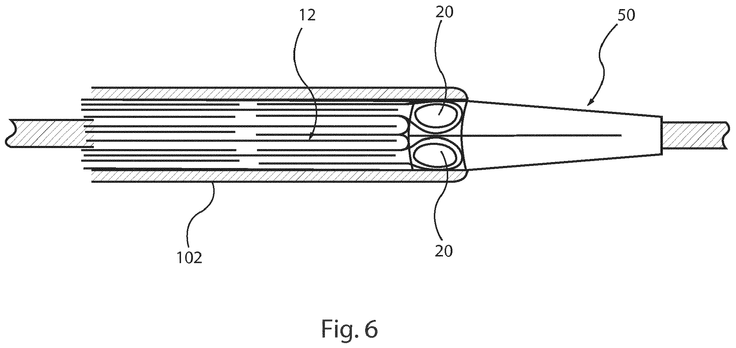

Referring now to Figures 5, 6 and 7 these show in schematic form and partially in cross-section the principal components of an introducer assembly 10 in which the medical device 10 is held. With reference first to Figures 5 and 6, the introducer assembly 100 includes an outer sheath 102, which may be of conventional form, and having a distal end 104 and a proximal end (not visible in the Figures) which is typically kept outside the patient, by the percutaneous entry point. The outer sheath 102 can be of any conventional form and may have a length from a few tens of centimetres to well over a metre, depending on the nature of the medical device carried by the introducer assembly and the percutaneous entry point of choice.

The assembly 100 also includes a pusher element 110, which in this embodiment is a piston head attached to a guide wire catheter 120. The guide wire catheter 120 also acts as a device support element supporting the medical device 10 when held in the introducer assembly 100. Typically, the guide wire catheter 120 would extend beyond the pusher piston head 110 towards the distal end 104 of the assembly 100, to terminate just short of or in abutment with the proximal face 54 of the guide tip 50. It will be seen in the embodiment of Figure 5 that the medical device 10 is provided with proximal radiopaque markers 20' as well as the distal radiopaque markers 20. It is typical for stents to be provided with radiopaque markers at least at both extremities.

As explained above, when the medical device 10 is radially compressed, as shown in Figures 5 to 7, the internal twist and sleeve 30 will loosen, allowing relatively unimpeded passage therethrough by the guide wire catheter 120. The lumen passing through the guide wire catheter 120 will in practice be aligned with the lumen within the guide tip 50, allowing passage of a guide wire 90 all the way through the assembly 100, enabling the assembly 100 to be delivered over the wire.

As will be apparent in particular from Figures 5 to 7, the guide tip 50 is designed to extend beyond the distal end 104 of the sheath 100 and to act as a dilator tip, in similar manner to a conventional dilator tip.

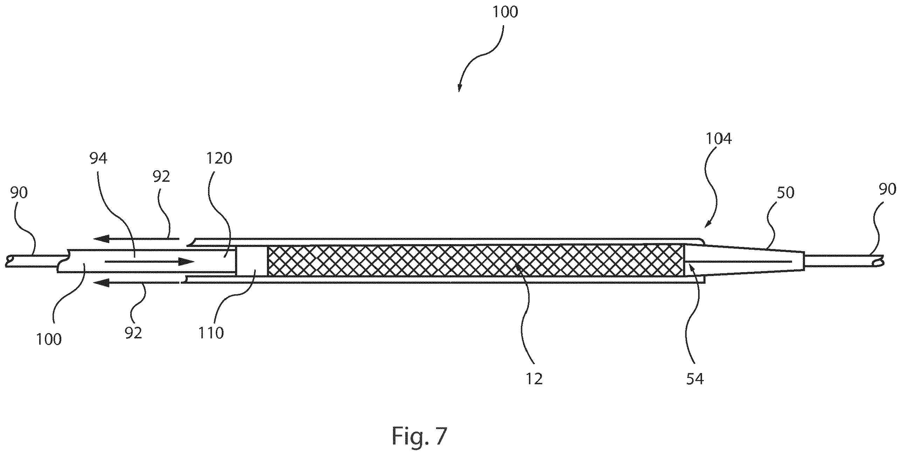

The medical device 10 can be withdrawn from the assembly 100 by relative movement of the sheath 100 in a proximal direction and the pusher head 110 in a distal direction, as depicted by the arrows 92, 94 shown in Figure 7. In practice, this may be achieved by pulling the sheath 100 backwards, in a proximal direction while holding the guide wire catheter 120 still, or by pushing the guide wire catheter 120 in a distal direction while holding the sheath 100 still, or by a combination of the two.

With reference now also to Figure 8, once the sheath 100 has been pulled back so as to expose the medical device 10, the latter will expand radially, typically under the natural spring force of the support structure 12, as shown in Figure 8. This radial expansion will cause the separation of the legs 62 of the guide tip 50 from their proximal ends, and its consequential longitudinal shortening. When the guide tip 50 expands radially in this manner, the support structure 12 will typically engage with the inner surfaces of the patient's vessel to become coupled thereto. In the case that the medical device 10 includes a plug element such as the sleeve 30 shown in Figure 1, this radial expansion will also cause tightening of the twisted portion 36 of the sleeve 30 around the guide wire catheter 120. However, and as will be apparent particularly from Figure 1, since the guide wire catheter 120 is substantially cylindrical, as is the guide wire 90, these can be pulled back through the twisted section 36 of the medical device 100 without difficulty, especially given the lack of any dilator tip normally provided at the distal end of the carrier portion of the guide wire catheter 120. The dilator tip 50 in this embodiment remains attached to the medical device is not pulled therethrough. Once the entirety of the assembly 100 has been removed from within the device, the twisted sleeve will close completely so as to plug the vessel. The structure therefore enables over-the-wire deployment of vascular plugs and other medical devices, and also enables the use of a dilator tip without restrictions which may be associated with conventional introducer dilators. The structure can be used not only for vascular plugs and filters but for any expandable medical device, with the advantage that the dilator tip does not need to be retracted through the device once deployed, with a consequential risk of snagging of the dilator tip against the internal parts of the medical device, potentially causing damage to the device or migration of the device as the dilator tip is pulled therethrough.

The guide tip 50 is preferably self-supporting and resilient, so that it will tend to a straight configuration, but can also bend around a guide wire as it passes through a curved vessels or into branch vessels, thereby assisting in the tracking of the introducer assembly 100 through curved vasculature.

It is envisaged that in some embodiments the guide tip may occlude fluid therethrough when in the expanded configuration, for instance when the medical device is a vascular plug. In such embodiments, the guide tip may be formed of an elastomeric material, having an expansion return force less than the expansion force of the frame of the medical device (for instance the stent), so as not to impinge on the opening of the medical device.

All optional and preferred features and modifications of the described embodiments and dependent claims are usable in all aspects of the invention taught herein. Furthermore, the individual features of the dependent claims, as well as all optional and preferred features and modifications of the described embodiments are combinable and interchangeable with one another.

The disclosures in British patent application number GB1619644.6, from which this application claims priority, and in the abstract accompanying this application are incorporated herein by reference.

## Claims

1. An implantable medical device having an expandable support structure, the support structure including a proximal end and a distal end;
a guide tip of flexible material and having a proximal end and a distal end, the guide tip including a plurality of sections extending from the guide tip distal end to the guide tip proximal end, the sections being attached to the distal end of the support structure and being radially expandable with the support structure, thereby allowing passage of fluid through the guide tip at least when the support structure is in an expanded condition and the sections radially expanded.

2. An implantable medical device according to claim 1, wherein the distal end of the guide tip retains a substantially constant diameter as the support structure is expanded.

3. An implantable medical device according to claim 1 or 2, wherein the sections of the guide tip are longitudinally separable from one another at least at the proximal end of the guide tip.

4. An implantable medical device according to claim 1 or 2, wherein the sections of the guide are longitudinally separate from one another at the proximal end of the guide tip and are connected together at the distal end of the guide tip.

5. An implantable medical device according to any preceding claim, wherein the guide tip includes a passage extending therethrough.

6. An implantable medical device according to any preceding claim, wherein the guide tip is self-supporting.

7. An implantable medical device according to any preceding claim, wherein the guide tip has a shape tapering from its proximal end to its distal end.

8. An implantable medical device according to claim 7, wherein the guide tip is at least partially frusto-conical.

9. An implantable medical device according to any preceding claim, wherein the guide tip is a dilator tip.

10. An implantable medical device according to any preceding claim, wherein the support structure is an expandable frame formed of a plurality of struts and the proximal end of the guide tip is attached to one or more the struts.

11. An implantable medical device according to any preceding claim, wherein the support structure is an expandable stent.

12. An implantable medical device according to any preceding claim, wherein the medical device is a vascular filter or occluder.

13. An implantable medical device according to claim 12, wherein the support structure supports a filter or occlusion member.

14. An implantable medical device according to any preceding claim, wherein the support structure is a self-expanding.

15. An introducer assembly including a carrier catheter having a proximal end and a distal end, and a lumen extending therethrough;
an implantable medical device having an expandable support structure, the support structure including a proximal end and a distal end;
a guide tip of flexible material and having a proximal end and a distal end, the guide tip including a plurality of sections extending from the guide tip distal end to the guide tip proximal end, the sections being attached to the distal end of the support structure and being radially expandable with the support structure, thereby allowing passage of fluid through the guide tip at least when the support structure is in an expanded condition and the sections radially expanded;
wherein the implantable medical device is disposable at the distal end of the carrier catheter with the guide tip at least partially extending beyond the distal end of the carrier catheter.

16. An introducer assembly according to claim 15, including an elongate carrier element, the implantable medical device being disposed on the elongate carrier element for insertion into the carrier catheter.

17. An introducer assembly according to claim 16, wherein the elongate carrier element is a guide wire catheter.

18. An introducer assembly according to claim 15, 16 or 17, including a protective sheath disposed over the implantable medical device, the guide tip being positionable beyond a distal end of the protective sheath to act as a dilator tip.
